# FASCICULE DE BREVET EUROPEEN

(11) **EP 0 748 871 B1**
(45) Date de publication et mention de la délivrance du brevet: **20.08.2003**
(21) Numéro de dépôt: 95201616.0
(22) Date de dépôt: 16.06.1995
(51) Int. Cl.: C12N 15/34, C12N 15/74, C12N 1/21, C12N 1/04

(54) **Streptococcus résistant aux phages**
Phagenresistenter Streptococcus
Phage-resistant streptococcus

(43) Date de publication de la demande: 18.12.1996
(73) Titulaire: SOCIETE DES PRODUITS NESTLE S.A., 1800 Vevey (CH)
(72) Inventeur: Mollet, Beat, CH-1074 Mollie-Margot (CH); Pridmore, David, CH-1000 Lausanne 26 (CH); Zwahlen, Marie Camille, CH-1018 Lausanne (CH)

(56) Documents cités:
- EP-A- 0 246 909
- EP-A- 0 474 464
- J. BACTERIOL. (1990), 172(11), 6419-26 CODEN: JOBAAY;ISSN: 0021-9193, 1990 HILL, COLIN ET AL 'Cloning, expression, and sequence determination of a bacteriophage fragment encoding bacteriophage resistance in Lactococcus lactis'
- FEMS MICROBIOL. LETT. , vol. 12, no. 11-3, Septembre 1993 pages 87-108, COLIN HILL 'Bacteriophage and bacteriophage resistance in lactic acid bacteria'

### Liste de séquence

### (1) INFORMATIONS GENERALES:

(i) DEPOSANT:
   (A) NOM: SOCIETE DES PRODUITS NESTLE
   (B) RUE: AVENUE NESTLE 55
   (C) VILLE: VEVEY
   (D) ETAT OU PROVINCE: CANTON DE VAUD
   (E) PAYS: SWITZERLAND
   (F) CODE POSTAL: 1800
   (G) TELEPHONE: (41).21.924.47.60
   (H) TELECOPIE: (41).21.924.28.80
(ii) TITRE DE L' INVENTION: STREPTOCOCCUS RESISTANT AUX PHAGES
(iii) NOMBRE DE SEQUENCES: 1
(iv) FORME DECHIFFRABLE PAR ORDINATEUR:
   (A) TYPE DE SUPPORT: Floppy disk
   (B) ORDINATEUR: IBM PC compatible
   (C) SYSTEME D' EXPLOITATION: PC-DOS/MS-DOS
   (D) LOGICIEL: Patentin Release #1.0, Version #1.30 (OEB)

### (2) INFORMATIONS POUR LA SEQ ID NO: 1:

(i) CARACTERISTIQUES DE LA SEQUENCE:
   (A) LONGUEUR: 856 paires de bases
   (B) TYPE: nucléotide
   (C) NOMBRE DE BRINS: double
   (D) CONFIGURATION: linéaire
(ii) TYPE DE MOLECULE: ADN (génomique)
(xi) DESCRIPTION DE LA SEQUENCE: SEQ ID NO: 1:
   GAATTCAAGG AAGAAAATAA CACAGTTTAT AAATTCCTTA ATGAATATTT GTCAGATGTC      60
   GTTTCCACTC GTATTCCAGT TAGATTCTTG TGGGATGTAT ACCGCTCATG GTGTCACGAG     120
   GGTAATCATA CTATCCCTAA AAAATCTAAC TTTGAAAAAG AGTTGGCACA GAATTTACCA     180
   GTTGGTTGGA TAAAAGATAG ACAAAAACCT CTTGATTTTT TTAACCCAAC TAAAGATAAG     240
   CCAGTTTATT GGCATGATTT CAATTTTAAT TGGGACGAAA ACGAGGCGAA GAAAGCAGCA     300
   GTAGTGGTTA TGGTTACTCA GTAACCGCAG GTTATTGCAA CAAGTAACCG TAAAACCCAT     360
   TGAAAATAAA GGGTTTCGGT TGCTTTAGTT ACTTAGTTAC TACTTTTAAA TATATTTATA     420
   AATAAATAAA TAAATAAATA AATATATATA GAGAGAGACT TAAAAAAACG TGTAACTAAG     480
   TAACTAAAGT GGCCAGAAAC CTTGATATAT AAGGGGTTTG CGGTGGTTAC GAGTAAAAGT     540
   AACTGTTACT GTAATCGAGT AACAAAAGGA GAAAAAAATG GAAATTCAAT ACTTAGAGAT     600
   TAATCAAGAA CACGAACCTA ATGAAAATAT TAGTAATTAC ATCAAAGATT TTTCTGAAGC     660
   GGCAACAGTT ATAGATGTGC AATGCAACGC TATTCCGGTA CATTTTGAAA AGGTTGGAGA     720
   AGACTATTGG ATCGATGAAG ATTATGGCAT TAAAGTTGTT GCGTTTATCA AATATGAAGA     780
   TAACAAAGAG GCAACTCCAG AAAAGAAACA ATGGTTGAAG GAATTCAGTC AGATGTCGTT     840
   TCCACTCGTA TTCCAG                                                     856

## Description

La présente invention se rapporte à l'utilisation d'un fragment d'ADN de bactériophage pour rendre résistante aux bactériophages une bactérie lactique le contenant.

### Etat de la technique

La vulnérabilité des bactéries lactiques à une attaque phagique représente un problème majeur dans l'industrie du yogourt qui traite des volumes importants de lait. Les bactériophages (ou phages) peuvent être apportés au cours du processus de fabrication du yogourt à cause d'un manque d'hygiène. Ils peuvent aussi apparaître suite à l'entrée en phase lytique d'un phage lysogène, c'est à dire un phage intégré dans le génome d'une bactérie sous une forme latente. Pour réduire l'incidence de ces attaques, l'industrie recours généralement à l'utilisation d'un mélange de souches différentes de bactéries lactiques naturellement résistantes à certains phages, ou à l'utilisation en rotation pour chaque étape de production à des souches différentes de bactéries lactiques. Cependant ces systèmes présentent l'inconvénient que l'on est obligé d'utiliser des souches différentes de bactéries lactiques, ce qui conduit à masquer les propriétés organoleptiques et de texture d'une souche intéressante par rapport aux autres souches utilisées. Il serait donc particulièrement avantageux de pouvoir avoir des moyens pour élargir le spectre de résistance d'une souche de bactérie lactique intéressante.

La résistance naturelle aux phages des bactéries lactiques est généralement d'origine bactérienne, et fait intervenir des mécanismes de restriction/modification, d'interférence à l'adsorption des particules phagiques, et/ou d'avortement de la phase lytique du phage. Ces résistances sont de plus généralement codées par un plasmide bactérien. Larbi et al. décrivent ainsi deux souches de *Streptococcus salivarius* subsp. *thermophilus* (*S. thermophilus*) présentant au moins trois mécanismes bactériens de résistance aux phages (J. Dairy Res., 59, 349-357, 1992).

Ces mécanismes bactériens de résistance aux phages peuvent être par ailleurs utilisés pour élargir le spectre de résistance aux phages des bactéries lactiques. Sing W. D. et al. décrivent ainsi la fabrication de clones de *Lactococcus lactis* subsp. *lactis* (*Lactococcus lactis*) comprenant chacun un plasmide différent codant pour un mécanisme bactérien de résistance aux phages (Applied and Environmental Microbiology, 59, 365-372, 1993).

Enfin, une autre source potentielle de mécanismes de résistance aux phages de bactéries lactiques a été récemment mise en évidence par Hill C. et al. (Journal of Bacteriology, 172, 6419-6426, 1990). Des fragments d'ADN d'un phage virulent contre des *Lactococcus lactis* peuvent en effet conférer une résistance aux phages à des *Lactococcus lactis* qui les contiennent. Les mécanismes de résistance impliqués ne sont pas encore clairs. On peut cependant raisonnablement estimer qu'ils sont liés à la surproduction ou la titration de signaux régulateurs essentiels au développement des phages. La connaissance scientifique de cette publication forme la base de EP 0 474 464.

### Résumé de l'invention

La présente invention a pour but de fournir de nouveaux fragments d'ADN de phages capables de conférer une résistance aux phages à un *Streptococcus.*

A cet effet, la présente invention concerne un fragment d'ADN de phages virulents contre un *Streptococcus* capable de conférer à un *Streptococcus* le contenant une résistance à au moins un phage, ledit fragment s'hybridant dans des conditions rigoureuses avec
le fragment HindIII de 3.6 kilo-bases (kb) présent dans le plasmide CNCMI-1588 ou le fragment EcoRV de 6.5 kb présent dans le plasmide CNCM I-1589.

L'invention concerne aussi un fragment d'ADN de phages virulents contre un *Streptococcus* capable de conférer à un *Streptococcus* le contenant une résistance à au moins un phage, ledit fragment présentant plus de 80% d'homologie ou s'hybridant avec le fragment ayant la séquence SEQ ID NO:1.

L'invention concerne aussi un procédé pour rendre résistant un *Streptococcus* à au moins un phage, caracterisé en ce que l'on clone dans un vecteur le fragment d'ADN selon la présente invention, et on introduit le vecteur dans un *Streptococcus.*

La présente invention concerne également les organismes du genre *Streptococcus* comprenant, intégrés dans leur génome ou par le moyen d'un plasmide réplicable, le fragment selon la présente invention.

Les vecteurs de réplication ou d'intégration recombinants comprenant un fragment d'ADN selon la présente invention, sont également un objet de l'invention.

Les *Streptococci* transformés par un fragment d'ADN selon l'invention présentent la surprenante propriété d'être résistants non seulement au phage à partir duquel le fragment d'ADN a été isolé, mais aussi aux phages qui leur sont homologues et mêmes à certains phages qui leur sont hétérologues.

Les fragments d'ADN selon la présente invention permettent donc d'élargir considérablement le spectre de résistance aux phages d'un *Streptococcus.* Pour cela, on peut transformer un *Streptococcus* par un vecteur comprenant un ou plusieurs fragments selon l'invention pouvant porter des mécanismes de résistance différents. On peut aussi transformer un *Streptococcus* par un vecteur selon l'invention comprenant en outre un ou plusieurs mécanismes bactérien de résistance.

D'une manière surprenante, le spectre de résistance conféré par un fragment selon l'invention peut être différent selon la souche de *Streptococcus* renfermant ledit fragment.

Finalement, on peut aussi envisager de développer des clones d'une souche de *Streptococcus* intéressante ayant chacun un spectre de résistance différent. Ces clones de *Streptococcus* pourraient être ainsi utilisés avantageusement en rotation lors de la fermentation industrielle d'un lait pour fabriquer du yogourt ou du fromage, par exemple.

### Description détaillée de l'invention

Dans la suite de la description, on entend par "phage homologue" les phages faisant parti d'un groupe de phages qui attaquent les mêmes souches de bactéries et qui ont un comportement lytique similaire. A l'inverse, un phage ne remplissant pas les deux conditions définies ci-dessus est un phage hétérologue.

Au sens de la présente invention, on entend par "séquence homologue" toute séquence ne différant des séquences selon l'invention que par la substitution, la délétion ou l'addition d'un petit nombre de bases. Dans ce cadre, on considérera en particulier comme homologue deux séquences d'ADN qui, du fait de la dégénérescence du code génétique, codent pour un même polypeptide. On considérera aussi comme séquence homologue, celle qui présente plus de 80% d'homologie avec les séquences selon l'invention. Dans ce dernier cas, l'homologie est déterminée par le rapport entre le nombre de bases d'une séquence homologue qui sont identiques à celles d'une séquence selon l'invention, et le nombre total de bases de ladite séquence selon l'invention.

Au sens de la présente invention, on entend par "fragment qui s'hybride" tout fragment capable de s'hybrider aux fragments selon l'invention par la méthode de Southern-Blot (Sambrook et al.., Molecular Cloning, A Laboratory Manual, Cold Spring Harbor Laboratory Press, U.S.A., 1989, chapitre 9.31 à 9.58). De préférence, l'hybridation est conduite dans des conditions rigoureuses de manière à éviter des hybridations aspécifiques ou peu stables.

Enfin, le terme "fragment" ou "fragment d'ADN" doit être compris comme un fragment d'ADN double brin d'origine phagique, qui peut être synthétisé, reproduit *in-vitro* par exemple par la méthode connue appelée "Polymérase Chain Reaction", ou reproduit *in-vivo* dans une bactérie du type *Escherchia coli* ou *Lactococcus lactis*, par exemple.

Pour mettre en oeuvre un procédé de sélection d'un fragment d'ADN selon l'invention, on peut fabriquer une banque de *Streptococci* renfermant des fragments d'ADN phagiques couvrant partiellement ou la totalité du génome d'un phage. Pour cela, on peut digérer de l'ADN phagique par une enzyme de restriction, on clone le produit de digestion dans un vecteur, puis on introduit les vecteurs dans des *Streptococci,* par exemple.

Ensuite on peut cultiver la banque de *Streptococci* transformés dans un milieu de culture comprenant des phages virulents contre des *Streptococci* de manière à séparer une deuxième banque de *Streptococci* résistants aux phages, par exemple.

On peut alors isoler classiquement des clones résistants aux phages en étalant des dilutions de la deuxième banque sur un milieu de culture solide, puis sélectionner parmi les clones isolés ceux qui présentent une résistance au moins 100 fois supérieure à celle du *Streptococcus* non-transformé, par exemple.

Comme alternative au procédé décrit ci-dessus, on peut aussi isoler préalablement chaque fragment d'ADN phagique par une électrophorèse d'un produit de digestion d'ADN phagique suivie d'une élution d'une bande de gel comprenant le fragment d'intérêt. Puis on peut cloner chaque fragment isolé dans un vecteur, introduire chaque vecteur dans un *Streptococcus,* et sélectionner un *Streptococcus* présentant une résistance au moins 100 fois supérieure à celle du *Streptococcus* non-transformé, par exemple.

Pour sélectionner parmi les *Streptococci* résistants ceux qui présentent une résistance au moins 100 fois supérieure à celle d'un *Streptococcus* non-transformé (par un fragment d'ADN phagique), on étale de préférence une dilution d'une suspension de phages virulent sur une culture confluente en milieu solide de *Streptococci* (transformés ou non), on compte le nombre de plaques (chaque plaque résultant d'une infection par un phage), et pour une dilution donnée on détermine le rapport entre le nombre de plaques apparaissant sur une culture de *Streptococci* non transformés et celui apparaissant sur une culture de *Streptococci* transformés. On peut ainsi sélectionner un *Streptococcus* transformé présentant un rapport d'au moins 100, de préférence un rapport compris entre 10³ et 10¹².

On a pu ainsi isoler du phage φSfi21 qui est particulièrement virulent contre des *S. thermophilus* (Collection du Centre de Recherche Nestlé, Lausanne) un fragment d'ADN HindIII de 3.6 kb. Ce fragment est présent dans le plasmide pMZ23 qui a été déposé, sous la forme de la souche *S. thermophilus* Sfi1 le contenant, le 7 juin 1995, auprès de la Collection Nationale de Culture de Microorganisme (C.N.C.M.), Institut Pasteur, 28 rue du Dr Roux, 75724 Paris cedex 15, France, où il a reçu le numéro de dépôt CNCM I-1588 . Ce fragment d'ADN HindIII de 3.6 kb permet de conférer à un *Streptococcus* qui le contient, notamment à un *S. thermophilus*, une résistance aux phages qui sont homologues au phage à partir duquel le fragment d'ADN a été sélectionné, mais aussi une résistance à certains phages qui lui sont hétérologues.

On a pu aussi isoler du phage φSfi2 qui est virulent contre des *S. thermophilus* (Collection du Centre de Recherche Nestlé, Lausanne) un fragment d'ADN EcoRV de 6.5 kb. Ce fragment est présent dans le plasmide pMZ31 déposé, sous la forme de la souche *S. thermophilus* Sfi1 le contenant, le 7 juin 1995, auprès de la C.N.C.M., Institut Pasteur, 28 rue du Dr Roux, 75724 Paris cedex 15, France, où il a reçu le numéro de dépôt CNCM I-1589. Il permet de conférer à un *Streptococcus* qui le contient, notamment à un *S. thermophilus*, une résistance à au moins un phage.

En particulier, on a pu isoler un fragment EcoRI de 0.8 kb qui est conservé dans les fragments susmentionnés HindIII et EcoRI. Ce fragment ayant la séquence SEQ ID NO:1 qui est donnée dans la liste de séquence ci-après, présente la particularité d'être relativement bien conservé dans les phages homologues au phage φSfi21. Il permet également de conférer à un *Streptococcus* qui le contient, notamment à un *S. thermophilus*, une résistance à au moins un phage.

Vu l'intérêt présenté par les fragments d'ADN de 6.5 kb, 3.6 kb et 0.8 kb, et le fait que pour la première fois des fragments d'ADN de phage virulent contre un *Streptococcus* ont pu conférer une résistance à un *Streptococcus* le contenant, la présente invention concerne tout fragment d'ADN de phages virulents contre un *Streptococcus* qui est capable de conférer à un *Streptococcus* une résistance à au moins un phage.

En particulier, l'invention peut concerner tout fragment d'ADN qui, premièrement est homologue ou qui s'hybride avec le fragment HindIII de 3.6 kb, le fragment EcoRV de 6.5 kb et/ou le fragment EcoRI de 0.8 kb, et deuxièmement est susceptible de conférer à un *Streptococcus* une résistance à au moins un phage. De préférence, on choisit des fragments d'une longueur d'au moins 20 paires de bases (pb), cette limite inférieure étant arbitrairement fixée du fait que les petits fragments s'hybridant spécifiquement ont généralement une longueur de 15-25 pb.

Pour mettre en oeuvre le procédé pour rendre résistant un *Streptococcus* à au moins un phage, on clone de préférence dans un vecteur un fragment homologue ou s'hybridant avec le fragment HindIII de 3.6 kb présent dans le plasmide CNCM I-1588 ou le fragment EcoRV de 6.5 kb présent dans le plasmide CNCM I-1589. De préférence, on utilise le fragment EcoRI de 0.8 kb.

Le vecteur peut être un plasmide d'expression réplicable pouvant comporter des systèmes de réplication et d'expression d'autres microorganismes, comme par exemple de *Escherichia coli* ou de *Lactococcus lactis*, par exemple. Il peut être aussi un vecteur d'intégration. Il faut également noter qu'il n'est pas utile de placer en amont de la séquence du phage, des séquences indispensables à l'expression de cette séquence, comme par exemple un promoteur de *Streptococcus.*

Dans le procédé d'expression ou de sélection selon la présente invention, on peut introduire le vecteur dans une bactérie du genre *Streptococcus,* notamment *S. thermophilus*, par conjugaison, transféction ou transformation, par exemple. L'organisme ainsi transformé comprend alors de préférence plusieurs copies du vecteur transformé.

La présente invention est décrite plus en détail ci-après à l'aide du complément de description qui va suivre, qui se réfère à des exemples d'obtention de fragments d'ADN, de plasmides recombinants et de bactéries transformées selon l'invention. Il va de soi, toutefois, que ces exemples sont donnés à titre d'illustration de l'objet de l'invention dont ils ne constituent en aucune manière une limitation.

La manipulation de l'ADN, le clonage et la transformation de cellules bactériennes sont, en l'absence de précisions contraires, effectués selon les protocoles décrits dans l'ouvrage de Sambrook et al. cité plus haut. Les pourcentages sont donnés en poids, sauf indication contraire.

### Figures

Figure 1: courbes de croissance en plaque de microtitration (densité optique en fonction du temps) de la souche de *S. thermophilus* Sfi1 transformée par le plasmide pNZ124 en présence ou en absence des phages φBas3, φBas11, φBas19, φSfi21, φSfi2 et φSfi18.
Figure 2: courbes de croissance en plaque de microtitration (densité optique en fonction du temps) de la souche de *S. thermophilus* Sfi1 transformée par le plasmide pMZ23 en présence ou en absence des phages φBas3, φBas11, φBas19, φBas3, φSfi21, φSfi2 et φSfi18.

### Milieux: (rajouter 1,5% de Bacto-agar pour un milieu solide)

- M17 (Difco,USA): 0,5% de tryptone, 0,5%, de soytone, 0,5% de viande hydrolysée, 0,25% d'extrait de levure, 0,05% d'acide ascorbique, 0,025% de sulphate de magnésium, 1,9% de disodium-beta-glycerophosphate, et de l'eau.
- LM17: milieu M17 comprenant 0,5% de lactose.
- LM17/CaCl₂: milieu LM17 comprenant 0,05% de CaCl₂.
- MSK: lait écrémé (poudre reconstitué à 10%) comprenant 0,1% d'extrait de levure.
- MRS: 1% de peptone, 1% de viande hydrolysée, 0,5% d'extrait de levure, 0,5% d'acétate de sodium, 0,01% de sulphate de magnesium, 0,2% de phosphate de dispotassium, 0,2% de citrate d'ammonium, 0,005% de sulphate de manganèse, 2% de dextrose, 0,1% de tween 80, et de l'eau.
- HJ: 3% tryptone, 0.2% extrait de boeuf, 1% extrait de levure, 0.4% de KH₂PO₄.

### Souches bactériennes:

- *S. thermophilus* Sfi21 qui a été déposée le 18 mai 1994, à la CNCM où il a reçu le numéro de dépôt CNCM I-1424. Cette souche est sensible au phage φSfi21 et naturellement résistante aux phages φBas3, φBas11, φBas19, φSfi2, φSfi9 et φSfi18.
- *S. thermophilus* Sfi1 qui est sensible aux phages φBas3, φBas11, φBas19, φSfi2 et φSfi18, et naturellement résistante au phage φSfi9. Deux clones de cette souche contenant le plasmide pMZ31 ou pMZ24 ont été déposés à la CNCM sous les numéros de dépôt cités ci-dessus CNCM I-1588 et CNCM I-1589.
- *S. thermophilus* Sfi9 qui a été déposée le 18 mai 1994 à la CNCM où il a reçu le numéro de dépôt CNCM I-1421. Cette souche est sensible au phage φSfi9 et naturellement résistante aux phages φBas11, φBas19, φBas3, φSfi2, φSfi18 et φSfi21.

Ces trois souches Gram-positifs présentent au microscope un aspect de coques formant des chaînettes non flagellés. Ces souches ne font pas de spores et elles sont anaéorobes facultatifs.

### Exemple I: clonage de fragments d'ADN du phage φSfi21

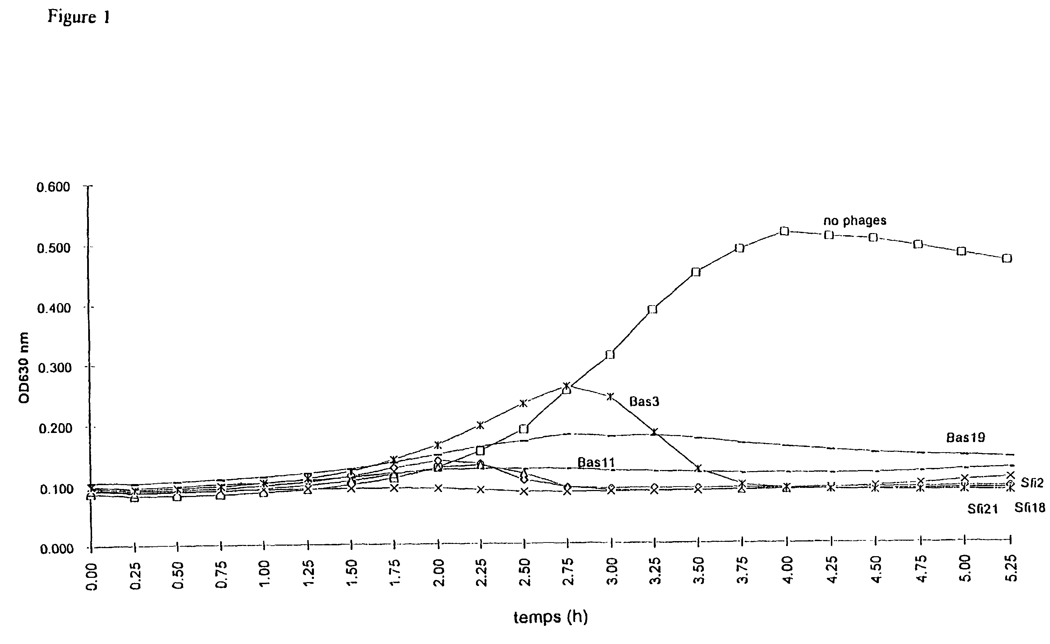

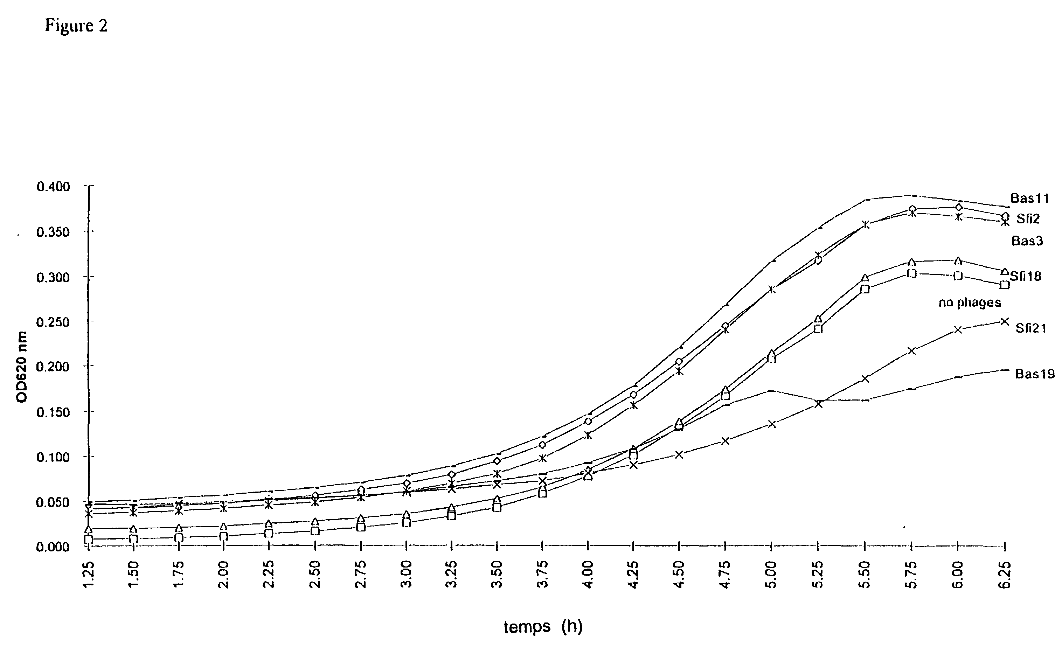

I.1. Préparation de fragments d'ADN du phage φSfi21: on inocule 200 ml d'un milieu liquide LM17/CaCl₂ avec 1% d'une culture en phase stationnaire de la souche *S. thermophilus* Sfi21 et 500µl d'une suspension de phage comprenant environ 10⁸ particules/ml (Collection Nestlé). On incube le milieu à 42°C jusqu'à ce que la lyse soit complète (environ 2 heures), on centrifuge le milieu pendant 30 min à 8000 rpm et à 4°C dans un rotor Sorval SS-34, on récupère le surnageant que l'on recentrifuge dans le même rotor pendant 6 h à 12000 rpm et à 4°C, puis on resuspend le culot de particules phagiques dans 0,5 ml d'un tampon comprenant 20mM Tris pH 7,2, 10 mM NaCl, 10 mM MgSO₄. On conserve alors la suspension de particules phagiques à -20°C (cette méthode peut être aussi appliquée pour préparer une suspension d'autres phages).
On traite la suspension de phages par 5µg/ml d'une désoxyribonucléase et 1µg/ml d'une ribonucléase pendant 30 min à 37°C, on ajoute 50 mM d'EDTA et 0.8% de SDS et on incube pendant 5 min à 37°C, puis on ajoute 100µg/ml de protéinase K et on incube 1h à 56°C. On extrait les protéines par 1 volume d'une solution de phénol saturée par du TE (100mM de Tris pH7,5 et 10mM d'EDTA), suivie par 1 volume de chloroforme. Puis on précipite l'ADN phagique présent dans la phase aqueuse dans 2 volumes d'éthanol comprenant 0,3M d'acétate de sodium pH5,2 à -70°C pendant au moins 30 min. On centrifuge à 13000rpm et on resuspend le culot d'ADN phagique dans 0,4 ml d'eau.
On digère ensuite classiquement une partie de la solution d'ADN phagique par l'enzyme de restriction HindIII (Boehringer-Mannheim, Germany) dans un tampon comprenant 20 mM de KCl, 10 mM de Tris-HCl pH8, 10 mM de MgCl₂, 1 mM de DTT et 0,1mg/ml de BSA. Puis dans les conditions décrites ci-dessus, on extrait les protéines au phénol, et on précipite et on resuspend l'ADN dans la solution de TE de manière à obtenir une solution comprenant 100ng/µl d'ADN phagique
I.2. Préparation d'une banque de *Streptococci* renfermant des fragments d'ADN couvrant une grande partie du génome du phage φSfi21: dans les conditions décrites ci-dessus, on digère classiquement le vecteur pNZ124 (de M. De VOS, Agricultural University of Wageningen, The Netherlands) par l'enzyme de restriction *Hind*III. A la fin de la réaction, on extrait les protéines au phénol, on précipite et on resuspend l'ADN dans la solution de TE de manière à obtenir une solution comprenant 100ng/µl d'ADN plasmidique.
On mélange une quantité de la solution de vecteur pNZ124 (100ng), une autre quantité de la solution de fragments d'ADN (100ng) et de l'eau jusqu'à 17µl. On chauffe le mélange pendant 2 à 5 min à 56°C, puis on ajoute 2µl d'un tampon classique de ligation et 1µl d'une solution de T4 DNA ligase (Boehringer Mannheim, Germany). A la fin de la réaction on extrait les protéines au phénol, puis on précipite classiquement l'ADN.
On prépare des cellules compétentes de la souche S. *thermophilus* Sfi1, sensible à tous les phages mentionnés dans les figures 1 et 2, par la méthode de Marciset et al., Biotechnology and Bioengineering, 43, 490-496, 1994.
On transforme par électroporation les cellules compétentes avec les vecteurs transformés (Marciset et al.). Pour cela, on mélange 200µl de la suspension de cellules compétentes décongelées avec le culot précipité de vecteurs recombinants, on place le mélange dans une cuvette d'un dispositif d'électroporation (Gen Pulser, Biorad), et on soumet la cuvette à 2100 V, 25µF et 400Ω. On ajoute ensuite 1ml du milieu de culture liquide HJ comprenant en outre 0,5% de lactose, et on incube à 42°C pendant 4h. On étale la culture sur un milieu LM17 solide comprenant en outre 4µg/ml de chloramphenicol. Les bactéries qui survivent constituent la banque de *Streptococci* renfermant des fragments d'ADN couvrant la totalité du génome du phage φSfi21. Il faut toutefois noter que certains *Streptococci* renferment le vecteur pNZ124 qui s'est recircularisé sans incorporer un ou plusieurs fragments phagiques.
I.3. Préparation d'une banque de *Streptococci* résistants aux phages: on cultive la banque de *Streptococci* ci-dessus dans un milieu de culture liquide LM17 comprenant en outre 4µg/ml de chloramphenicol et 100µl de la suspension de particules de phages φSfi21 (point I.1 ci-dessus). Les bactéries qui survivent constituent la banque de *Streptococci* résistants aux phages.
I.4 Analyse du plasmide pMZ23: parmi les *S. thermophilus* résistants aux phages on a pu isoler un clone renfermant le plasmide pMZ23 qui a reçu le numéro de dépôt CNCM I-1588 et qui contient un fragment d'ADN phagique HindIII de 3.6 kb.
La résistance aux phages conférée par le plasmide pMZ23 peut être déterminée par un test en plaque de microtitration. Pour cela, on cultive le clone de *S. thermophilus* Sfi1 renfermant le plasmide pMZ23 dans du milieu MSK supplémenté de 4µg/ml de chloramphénicol. On dilue 100µl de la culture en phase stationnaire à 0,9ml de milieu LM17/CaCl₂ supplémenté de 4µg/ml de chloramphénico. On réparti dans les puits de la plaque de microtitration (en triple; dilution 1/100) 200µl du milieu ci-dessus, 22µl de la culture diluée et 2,5µl/ml de la suspension de phage φSfi21 ci-dessus. On incube la plaque à 42°C et on mesure la densité optique à 620-630nm toutes les 15 min pendant 7,5 h (Automatic Reader, Dynatech).
Le même test a été réalisé avec des phages homologues du phage φSfi21, c'est à dire les phages φSfi2, φSfi18, et φBas3, et des phages hétérologues du phage φSfi21, c'est à dire les phages φBas11 et φBas19. Pour cela, on a préparé une suspension de ces phages comme décrit au point I.1 ci-dessus.
Pour comparaison, on réalise en parallèle le même test avec la souche *S. thermophilus* Sfi1 transformée en absence de phages. On réalise aussi le même test avec la souche *S. thermophilus* transformée par le plasmide pNZ124.
Les résultats des essais présentés à la figure 1 montrent que la souche *S. thermophilus* Sfi1 transformée par le plasmide pNZ124 est sensible à tous les phages utilisés. Le plasmide pNZ124 n'est donc pas responsable de la résistance.
Les résultats des essais présentés à la figure 2 montrent que la souche *S. thermophilus* Sfi1 transformée par le plasmide pMZ23 est résistante à tous les phages utilisés. Le fragment phagique HindIII de 3.6 kb est donc bien responsable de la résistance. Il permet de conférer une résistance aux phages homologues et aux phages hétérologues du phage φSfi21.
I.5. Evaluation du degré de résistance: on mélange à 0,3ml d'une culture fraîche dans du MSK (supplémenté de 4µg/ml de chloramphénicol) des souches *S. thermophilus* Sfi1 transformées par les plasmides pNZ124 ou pMZ23, 2,5ml de milieu MRS agar (0,7% Bacto-agar) à 52°C, et 0,1ml de dilutions de la suspension de chaque phage évalué (les suspensions de phages sont préparées comme décrit au point I.1.). On étale le mélange sur un milieu solide LM17 supplémenté avec du chloramphenicol, on incube à 42°C sous anaérobiose, et on compte le nombre de foyers d'infection sur le milieu solide (sous la forme de plaques). On détermine ensuite pour une dilution donnée le rapport pNZ124/pMZ23 déterminé par le rapport entre le nombre de plaques sur une culture de *S. thermophilus* transformée par pNZ124 et celui sur une culture de *S. thermophilus* transformée par pMZ23.
Les résultats présentés dans le tableau 1 ci-après montrent que le fragment HindIII de 3.6kb du génome du phage φSfi21 permet de conférer une résistance aux phages qui sont homologues au phage φSfi21.

**Tableau 1**

| Phages virulents contre *S. thermophilus* Sfi1 | Rapport pNZ124 / pMZ23 |
|---|---|
| φSfi21 | 10⁵-10⁷ |
| φSfi 2 (homologue φSfi21) | >10⁷ |
| φSfi18 (homologue φSfi21) | >10⁷ |

I.6. Analyse du fragment HindIII de 3.6 kb : dans le but de déterminer si des parties du fragment HindIII de 3.6 kb pourraient également conférer une résistance aux phages, on clone des fragments EcoRI du fragment HindIII de 3.6 kb dans pNZ124 et on transforme le plasmide dans *S.thermophilus* Sfi1.
Pour cela, on purifie le plasmide pMZ23 et on le coupe en fragments par l'enzyme de restriction *Hind*III par des moyens connus de l'homme du métier. Les fragments d'ADN sont alors séparés par électrophorèse sur un gel d'agarose. On découpe du gel la bande comprenant le fragment de 3.6 kb, on dépose la bande sur une membrane de centrifugation de 0,45µm (MC ultrafree filter) que l'on conserve à -20°C pendant 10 min, et on centrifuge le tout pendant 20 min à 10000 rpm et à température ambiante dans une centrifugeuse Heraeus Biofuge A. On extrait le filtrat avec du phénol saturé de TE, puis on précipite la phase aqueuse par de l'acétate de sodium et de l'éthanol à -70°C pendant au moins 30 min. On centrifuge à 13000rpm, on resuspend le culot dans 0,5 ml d'une solution à 70% d'éthanol, on recentrifuge à 13000rpm, puis on resuspend le fragments d'ADN de 3.6 kb dans 10 µl d'eau.
On coupe le fragment de 3.6 kb par l'enzyme de restriction EcoRI, on ligue les fragments au plasmide pNZ124 préalablement coupé par *Eco*RI, et on transforme les plasmides recombinants à la souche *S. thermophilus* Sfi1. Les conditions expérimentales sont similaires à celles présentées au point I.1 et I.2 ci-dessus. On sélectionne ensuite les bactéries résistantes aux phages φSfi21 dans des conditions similaires à celles présentées au point I.3 ci-dessus, et on isole un clone particulièrement résistant renfermant un plasmide pMZ23c comprenant un fragment d'ADN phagique EcoRI de 0.8 kb.
On évalue la résistance conférée par ce fragment à un *Streptococcus* dans les conditions présentées au point I.4 ci-dessus. Les résultats présentés dans le tableau 2 ci-après montrent que le fragment de 0.8 kb du génome du phage φSfi21 permet de conférer une résistance aux phages qui sont homologues au phage φSfi21.

**Tableau 2**

| Phages virulents contre *S. thermophilus* Sfi1 | Rapport pNZ124 / pMZ23c |
|---|---|
| φSfi21 | 10⁵ |
| φSfi2 (homologue φSfi21) | 10³ |
| φSfi18 (homologue φSfi21) | >10¹⁰ |

I.7 Séquencage du fragment EcoRI de 0.8 kb: on purifie le plasmide pMZ23c par des moyens connus de l'homme du métier, puis on séquence le fragment EcoRI de 0.8 kb par la méthode connue utilisant des didéoxynucléotides (Sanger et al, Proc. Natl. Acad. Sci., 74, 5463-5467, 1977). Pour cela, on utilise des oligonucléotides servant d'amorce qui s'hybrident aux séquences provenant du plasmide pNZ124, de part et d'autre de la séquence phagique.
Les résultats montrent que le fragment EcoRI de 0.8 kb présente la séquence SEQ ID NO:1 donnée dans la liste de séquence ci-après.

### Exemple II Clonage du fragment d'ADN EcoRV de 6.5 kb du phage φSfi2

On prépare une banque de souches *S.thermophilus* Sfi1 renfermant des plasmides pNZ124 rencombinants comprenant des fragments EcoRV du génome du phage φSfi2. On utilise pour cela une méthode similaire à celle décrite aux points I.1 et I.2 ci-dessus.

On prépare ensuite une banque de souches *S.thermophilus* Sfi1 résistantes aux phages, comme décrit au point I.3 ci-dessus. Parmi les *S. thermophilus* résistants aux phages, on a pu isoler un clone renfermant le plasmide pMZ31 qui a reçu le numéro de dépôt CNCM I-1589 et qui contient un fragment d'ADN phagique EcoRV de 6.5 kb.

La résistance aux phages conférée par le plasmide pMZ31 peut être aussi déterminée par des tests similaires à ceux décrits aux points I.4 et I.5 ci-dessus.

Des parties du fragment EcoRV de 6.5 kb peuvent également conférer une résistance aux phages à un *Streptococcus* le contenant. Pour cela, par une méthode similaire à celle décrite au point I.6. ci-dessus, on purifie le plasmide pMZ31, on le coupe en fragments à l'aide de diverses enzymes de restriction, on sous-clone ces fragments dans le plasmide pNZ124, et on transforme *S.thermophilus* Sfi1 avec les vecteurs recombinants (que l'on nomme pMZ31-fragment). Finalement on sélectionne des bactéries transformées présentant une résistance aux phages au moyen du test décrit au point I.5 ci-dessus. En particulier, on sélectionne des bactéries présentant un rapport "pNZ124/pMZ31-fragment" supérieur à 100. Ce rapport est déterminé par le nombre de plaques sur une culture de *S. thermophilus* transformée par pNZ124 et celui sur une culture de *S. thermophilus* transformée par pMZ31-fragment.

Les résultats présentés dans le tableau 3 ci-après montrent que quatre fragments issus du fragment EcoRV de 6.5 kb peuvent conférer une très bonne résistance à un *Streptococcus*, à savoir les fragments EcoRV-BglII de 3 kb, BglII-HIII de 2 kb, BglII-EcoRV de 3.5 kb et EcoRI-EcoRI de 0.8 kb.

**Tableau 3**

| Fragment d'ADN phagique dans *S. thermophilus* Sfi1 | Phage(s) utilisé(s) pour évaluer le degré de résistance (voir point I.6 ci-dessus) | Rapport pNZ124/pMZ31-fragment (voir point I.6 ci-dessus) |
|---|---|---|
| EcoRV-EcoRV de 6.5 kb | φSfi2 | 300 |
| EcoRV-BglII de 3 kb | φSfi2 | 10⁸ |
| | φSfi18 | >10¹⁰ |
| | φSfi21 | 10⁷ |
| BglII-HIII de 2 kb | φSfi2 | 2,6x10⁵ |
| BglII-EcoRV de 3.5 kb | φSfi2 | 3x10⁶ |
| EcoRI-EcoRI de 0.8 kb | φSfi2 | >10⁷ |
| | φSfi18 | >10¹⁰ |
| | φSfi21 | >10⁷ |

On peut remarquer que la résistance conférée à *S. thermophilus* Sfi1 peut être différente entre les fragments, même si ceux-ci présentent des parties identiques de séquences nucléiques. En effet, le fragment BglII-RcoRV de 3.5 kb contient le fragment BglII-HIII de 2 kb, et le fragment EcoRV-BglII de 3 kb contient le fragment EcoRI-EcoRI de 0.8 kb.

De plus, le séquencage du fragment EcoRI-EcoRI de 0.8 kb par la méthode des didéoxynucléotides révèle une séquence identique à la séquence SEQ ID NO:1. La souche *S. thermophilus* Sfi1 contenant le plasmide pMZ31-[EcoRI-EcoRI de 0.8 kb], soumise aux attaques des phages décrits à la figure 2, montre des courbes de croissance très similaires à celles obtenues dans les mêmes conditions pour la souche *S. thermophilus* Sfi1 contenant le plasmide pMZ23c.

### Exemple III

On transforme par électroporation le plasmide pMZ23 à la souche *S. thermophilus* Sfi9. On utilise pour cela une méthode similaire à celle décrite au point I.2 ci-dessus. L'évaluation du spectre de résistance de la souche transformée par un test similaire à celui décrit au point I.5 ci-dessus, montre que la souche *S.thermophilus* Sfi9 transformée est résistante au phage φSfi9 qui est un phage hétérologue des phages φSfi21 et φSfi2.

### Exemple IV

On prépare un starter congelé des souches *S. thermophilus* Sfi1 et *S. thermophilus* Sfi1 transformée par pMZ23, en inoculant un lait reconstitué stérile à raison de 1% d'une préculture (en milieu MSK) de chaque souche prise au stade de coagulation du milieu, en incubant le lait à une température d'environ 42°C jusqu'à un pH de 5,1, en le refroidissant jusqu'à une température de 4°C, puis en le congelant à -75°C.

On prépare des yogourts étuvés par inoculation directe en mélange des starters congelés. Pour cela, on ensemence un lait reconstitué pasteurisé (3,7% de matière grasse et 2,5% de poudre de lait écrémé) par 6ml de chaque starter congelé, on l'incube jusqu'à un pH d'environ 4,65, puis on le refroidit jusqu'à 4°C.

## Revendications

1. Fragment d'ADN de phages virulents contre un *Streptococcus* capable de conférer à un *Streptococcus* le contenant une résistance à au moins un phage, ledit fragment s'hybridant dans des conditions rigoureuses avec
le fragment HindIII de 3.6 kb présent dans le plasmide CNCM I- 1588 ou
le fragment EcoRV de 6.5 kb présent dans le plasmide CNCM I-1589.

2. Fragment d'ADN de phages virulents contre un *Streptococcus* capable de conférer à un *Streptococcus* le contenant une résistance à au moins un phage, ledit fragment présentant plus de 80% d'homologie ou s'hybridant avec le fragment ayant la séquence SEQ ID NO:1.

3. Fragment d'ADN selon l'une des revendications 1 et 2, constitué du fragment HindIII de 3.6 kb présent dans le plasmide CNCM I-1588, du fragment EcoRV de 6.5 kb présent dans le plasmide CNCM I-1589 ou du fragment ayant la séquence SEQ ID NO:1.

4. Procédé pour rendre résistant un *Streptococcus* à au moins un phage, **caractérisé en ce que** l'on clone dans un vecteur le fragment d'ADN selon l'une des revendications 1 à 3, et on introduit le vecteur dans un *Streptococcus.*

5. *Streptococcus* ayant intégré dans son génome ou par le moyen d'un plasmide réplicable le fragment d'ADN selon l'une des revendications 1 à 3.

6. Vecteur de réplication ou d'intégration recombinant comprenant un fragment d'ADN selon l'une des revendications 1 à 2.

7. Vecteur selon la revendication 6, constitué du plasmide CNCM I-1588 ou du plasmide CNCM I-1589.

## Claims

1. DNA fragment of virulent phages against a *Streptococcus*, capable of conferring, to a *Streptococcus* containing it, resistance to at least one phage, said fragment hybridizing under rigorous conditions with
the 3.6 kb HindIII fragment present in plasmid CNCM I-1588 or
the 6.5 kb EcoRV fragment present in plasmid CNCM I-1589.

2. DNA fragment of virulent phages against a *Streptococcus,* capable of conferring, to a *Streptococcus* containing it, resistance to at least one phage, said fragment having a homology of more than 80% with, or hybridizing with, the fragment having the sequence SEQ ID NO:1.

3. DNA fragment according to Claim 1 or 2 consisting of the 3.6 kb HindIII fragment present in plasmid CNCM I-1588, the 6.5 kb EcoRV fragment present in plasmid CNCM I-1589 or the fragment having the sequence SEQ ID NO:1.

4. Method of rendering a *Streptococcus* resistant to at least one phage, **characterized in that** the DNA fragment according to one of Claims 1 to 3 is cloned into a vector and the vector is introduced into a *Streptococcus.*

5. *Streptococcus* having the DNA fragment according to one of Claims 1 to 3 integrated in its genome or via a replicatable plasmid.

6. Recombinant replication or integration vector comprising a DNA fragment according to Claim 1 or 2.

7. Vector according to Claim 6 consisting of plasmid CNCM I-1588 or plasmid CNCM I-1589.

## Patentansprüche

1. DNA-Fragment von gegenüber einem *Streptococcus* virulenten Phagen, das einem es enthaltenden *Streptococcus* eine Resistenz gegenüber mindestens einem Phagen verleihen kann, wobei dieses Fragment mit dem
Fragment HindIII von 3,6 kb, das in dem Plasmid CNCM I-1588 vorliegt, oder mit dem
Fragment EcoRV von 6,5 kb, das in dem Plasmid CNCM I-1589 vorliegt,
bei stringenten Bedingungen hybridisiert.

2. DNA-Fragment von gegenüber einem *Streptococcus* virulenten Phagen, das einem es enthaltenden *Streptococcus* eine Resistenz gegenüber mindestens einem Phagen verleihen kann, wobei dieses Fragment mehr als 80% Homologie mit dem die Sequenz SEQ ID NO:1 aufweisenden Fragment besitzt oder mit diesem hybridisiert.

3. DNA-Fragment nach einem der Ansprüche 1 und 2, bestehend aus dem im Plasmid CNCM I-1588 vorliegenden Fragment HindIII von 3,6 kb, aus dem im Plasmid CNCM I-1589 vorliegenden Fragment EcoRV von 6,5 kb oder aus dem Fragment mit der Sequenz SEQ ID NO:1.

4. Verfahren zur Herstellung einer Resistenz eines *Streptococcus* gegenüber mindestens einem Phagen, **dadurch gekennzeichnet, daß** man das DNA-Fragment nach einem der Ansprüche 1 bis 3 in einen Vektor klont und den Vektor in einen *Streptococcus* einführt.

5. *Streptococcus*, der das DNA-Fragment nach einem der Ansprüche 1 bis 3 in sein Genom oder mit Hilfe eines replizierbaren Plasmids integriert hat.

6. Rekombinanter Replikations- oder Integrationsvektor, der ein DNA-Fragment nach einem der Ansprüche 1 bis 2 umfaßt.

7. Vektor nach Anspruch 6, bestehend aus dem Plasmid CNCM I-1588 oder aus dem Plasmid CNCM I-1589.
